# FASCICULE DE BREVET EUROPEEN

(11) **EP 3 007 734 B1**
(45) Date de publication et mention de la délivrance du brevet: **11.08.2021**
(21) Numéro de dépôt: 14749861.2
(22) Date de dépôt: 10.06.2014
(51) Int. Cl.: A61L 2/10, A61L 2/22

(54) **DISPOSITIF DE DÉCONTAMINATION POUR MATÉRIEL MÉDICAL**
DEKONTAMINATIONSVORRICHTUNG FÜR EIN MEDIZINISCHES MATERIAL
DECONTAMINATION DEVICE FOR MEDICAL MATERIAL

(30) Priorité: 10.06.2013 FR 1355335
(43) Date de publication de la demande: 20.04.2016
(73) Titulaire: L.B.A. Consulting, 89170 Saint-Fargeau (FR)
(72) Inventeur: BARREAU, Christophe, 89520 Treigny (FR); BERTRAND, Eric, 89250 Seignelay (FR); MACAIRE, Philippe, Dubai (AE); CARPRIEAUX, Jean-Paul, 95450 Avernes (FR)
(74) Mandataire: Cabinet Chaillot
(86) Numéro de dépôt international: PCT/FR2014/051375
(87) Numéro de publication internationale: WO 2014/199058

(56) Documents cités:
- US-A- 4 029 967
- US-A1- 2004 091 389
- US-A1- 2009 020 135
- US-A1- 2012 107 184
- None

## Description

### Domaine technique

L'invention concerne le domaine de la décontamination des dispositifs médicaux et préférentiellement la décontamination des manches de laryngoscopes.

### Technique antérieure

Les dispositifs médicaux sont disponibles sous formes jetables ou réutilisables. Il est particulièrement intéressant d'utiliser du matériel jetable pour garantir une asepsie optimale et empêcher la transmission de micro-organismes d'un patient à l'autre. Toutefois, tous les dispositifs médicaux ne peuvent pas être produits sous forme de matériel jetable. De plus, l'absence d'étanchéité de certains de ces dispositifs et la présence de batteries ne permet pas de les nettoyer par immersion.

Le laryngoscope est un instrument médical servant principalement à l'intubation trachéale en permettant de visualiser la glotte. Il est composé de deux parties :
- une lame qui sert à dégager la langue, le palais mou et l'épiglotte, cette lame peut-être à usage unique,
- un manche servant à manipuler l'instrument. Il contient les batteries et, dans le cas des laryngoscopes à fibre optique, la source lumineuse. Cette portion du laryngoscope est réutilisable et doit être nettoyée entre chaque utilisation.

Selon un article du British Journal of Anaesthesia (Young et al. Novembre 2011), il est recommandé que les poignées de laryngoscope soient stérilisées entre chaque utilisation, mais seulement 22% des services hospitalier autoclavent les manches de laryngoscope entre chaque utilisation. Actuellement, il n'existe aucun protocole clair pour cette décontamination. Dans cette étude 40% des poignées de laryngoscope « propre » analysées étaient contaminées par des microbes.

Habituellement, la méthode de décontamination consiste à utiliser des lingettes jetables imprégnées d'un liquide de décontamination avant et après chaque utilisation du manche de laryngoscope. Toutefois, cette méthode n'est pas totalement satisfaisante puisqu'elle est dépendante de l'opérateur qui pratique la décontamination et qu'elle est particulièrement longue. De plus, les manches de laryngoscope présentent des aspérités dans lesquelles des fibres peuvent être retenues et qui sont difficiles à nettoyer en profondeur.

Une étude menée par les déposants a pu mettre en évidence que sur un échantillon de 25 manches de laryngoscope, 4 étaient toujours contaminés après la procédure habituelle de décontamination. Parmi les contaminants isolés on peut citer le staphylocoque doré, le bacille pyocyanique, Klebsiella Pneumoniae et E. Coli.

La présente invention concerne un dispositif permettant la décontamination des dispositifs médicaux et plus particulièrement des manches de laryngoscope. La présente invention permet de résoudre les problèmes liés aux méthodes de l'art antérieur. Plus particulièrement, le dispositif selon l'invention permet une décontamination rapide, automatisée et totale des dispositifs médicaux.

Le brevet américain US 2012/0107184 décrit un dispositif de décontamination tel que divulgué dans le préambule des revendications 1 et 2.

### Résumé de l'invention

Ainsi, la présente invention concerne un dispositif de décontamination pour matériel médical comprenant un support destiné à recevoir et à maintenir ledit matériel médical à décontaminer selon un axe prédéfini, le dispositif étant tel que défini à l'une quelconque des revendications 1 et 2.

Dans le cadre de la présente invention, le terme "axe prédéfini" entend signifier que lorsque le matériel médical à décontaminer est placé sur ledit support il est maintenu selon une position sensiblement constante d'une utilisation à l'autre et occupe sensiblement l'espace d'un cylindre dont l'axe de symétrie coïncide avec ledit « axe prédéfini ». Ainsi, ledit « axe prédéfini » correspond à l'axe de symétrie longitudinal du volume intérieur délimité par le moyen d'aspersion, le moyen de séchage et le moyen d'irradiation.

Dans le cadre de la présente invention, « monté en rotation autour dudit axe prédéfini » entend signifier que les moyens en question sont mobiles en rotation autour dudit axe prédéfini et/ou que ledit support destiné à recevoir et à maintenir ledit matériel médical à décontaminer selon un axe prédéfini est monté en rotation autour dudit axe prédéfini et que lesdits moyens en question sont fixes.

Dans le cadre de la présente invention, le terme « parallèlement audit axe prédéfini » entend signifier que les moyens en question sont mobiles en translation parallèlement audit axe prédéfini et/ou que ledit support destiné à recevoir et à maintenir ledit matériel médical à décontaminer selon un axe prédéfini est monté en translation selon ledit axe prédéfini et que les moyens en question sont fixes.

Dans le cadre de la présente invention, le terme « moyen d'aspersion » fait référence aux moyens capables de projeter un liquide.

Dans le cadre de la présente invention, le terme « moyen d'irradiation » fait référence aux sources de rayonnement de longueurs d'ondes comprises entre 150 et 450nm.

Selon un mode de réalisation préféré de l'invention, ledit moyen d'aspersion, ledit moyen de séchage et ledit moyen d'irradiation sont compris dans une pièce formant un passage cylindrique, destiné à recevoir ledit matériel médical à décontaminer. Dans ce cas, ledit « axe prédéfini » est l'axe de symétrie longitudinale dudit cylindre.

Selon l'invention ledit moyen d'aspersion, ledit moyen de séchage et ledit moyen d'irradiation sont compris dans une ou deux pièces, disposés selon le même axe longitudinal, formant un passage cylindrique, destiné à recevoir ledit matériel médical à décontaminer. Dans ce cas, ledit « axe prédéfini » est l'axe de symétrie longitudinale dudit cylindre.

Selon un mode de réalisation préféré de l'invention, ledit moyen d'irradiation forme un cylindre disposé sur la surface interne de la pièce dans laquelle il est compris.

Selon un mode de réalisation préféré de l'invention, ledit moyen de séchage est également monté en rotation autour de l'axe prédéfini.

Selon un mode de réalisation préféré de l'invention, ledit moyen d'irradiation est également monté en rotation autour de l'axe prédéfini.

Selon l'invention, ledit moyen de séchage est un moyen capable de produire une lame d'air.

Selon un mode de réalisation préféré de l'invention, ledit moyen capable de produire une lame d'air est disposé annulairement autour de l'axe prédéfini.

Dans ces trois derniers modes de réalisation, l'utilisation d'une lame d'air permet de sécher le dispositif mais également de décoller les matières solides éventuellement placées à sa surface. Ceci permet d'arriver à des niveaux de décontamination largement supérieurs à ceux précédemment observés.

Selon un mode de réalisation préféré de l'invention, ledit moyen d'irradiation est une source d'UV et préférentiellement d'UV-C.

Selon un autre mode de réalisation tout à fait préféré, ledit moyen d'irradiation est une source capable d'émettre séquentiellement ou simultanément des rayonnements appartenant à la gamme des UV-A, des UV-B et des UV-C.

Selon un mode de réalisation préféré de l'invention, ledit moyen d'aspersion est un moyen de nébulisation.

Dans le cadre de la présente invention, le terme « moyen de nébulisation » entend désigner tout moyen permettant de transformer une solution liquide en un brouillard de particule en suspension dans un gaz.

### Brève description des dessins

La figure 1 présente une vue de face d'un mode de réalisation d'un dispositif selon l'invention.
La figure 2 présente une vue en coupe du même mode de réalisation.

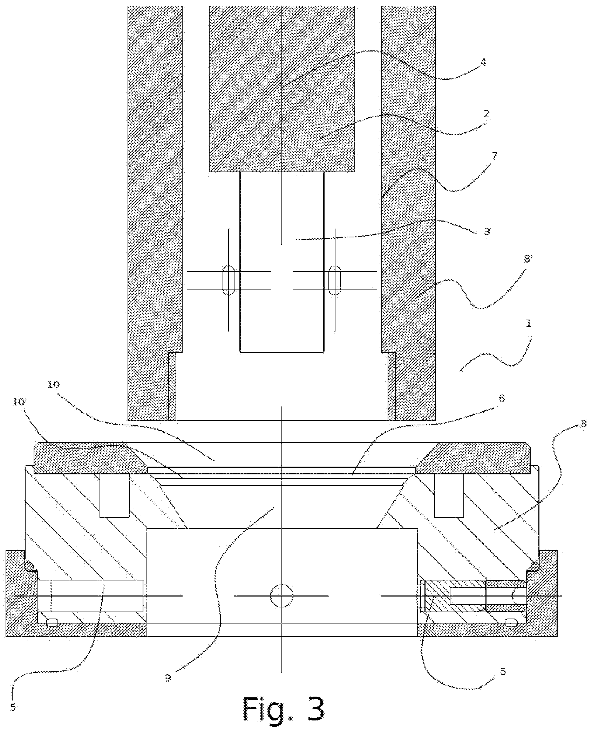

La figure 3 présente une vue partielle en coupe du même mode de réalisation.

### Description des modes de réalisation

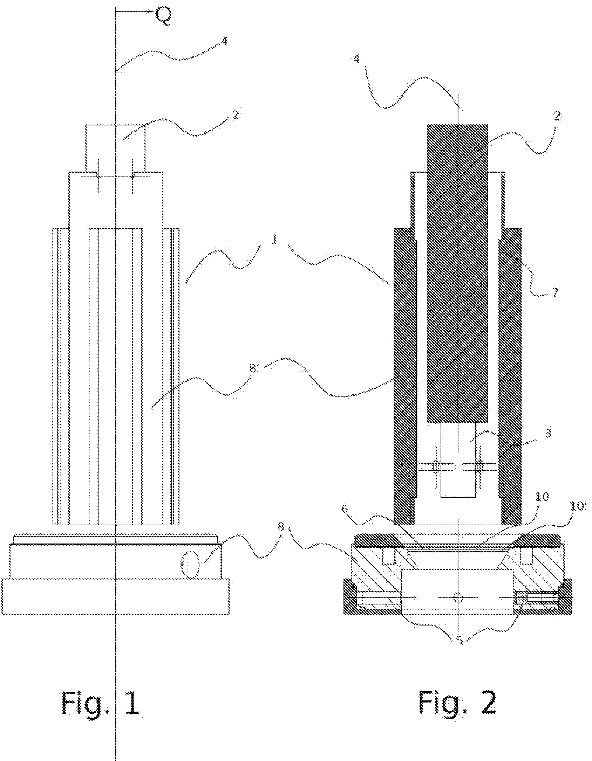

En référence aux figures 1 et 2, le dispositif 1 est constitué d'une colonne verticale, comprenant les différents moyens 5, 6, 7 permettant la décontamination du matériel médical 2.

Selon un mode de réalisation préféré, ledit matériel médical 2 est choisi dans le groupe comprenant les manches de laryngoscopes et les sondes.

Le dispositif 1 selon l'invention est préférentiellement placé dans une enceinte pouvant être totalement fermée. Ladite enceinte comporte préférentiellement une trappe d'accès permettant de placer le matériel médical 2 sur le support 3 et de le retirer dudit support 3 à l'issue de la procédure de décontamination.

Le support 3 est préférentiellement associé à un moyen permettant le déplacement dudit support 3 en translation et en rotation par rapport à l'axe prédéfini 4. L'ensemble des moyens compris dans le dispositif 1 selon l'invention sont préférentiellement commandé automatiquement par un dispositif de commande avantageusement programmable (e.g. carte électronique, ordinateur).

Le support 3 comprend préférentiellement un moyen permettant d'associer le support et le matériel à décontaminer 2. Ce moyen peut prendre toutes formes, parmi les moyens préférés, on peut citer les tiges filetées. Encore plus préférentiellement, ledit moyen est identique au moyen permettant d'associer le manche de laryngoscope et la lame destinée à être utilisée avec ledit manche.

Une fois que le matériel à décontaminer 4 est fixé sur le support 3, l'enceinte est close et le processus de décontamination est mis en route par l'opérateur.

Le matériel à décontaminer 2 va se déplacer verticalement avec une rotation comprise entre 45° et 360°. Alternativement, ce sont les moyens permettant la décontamination 5, 6, 7 qui vont se déplacer et le matériel médical à décontaminer 2 reste fixe.

Dans un premier temps, le dispositif médical va se déplacer dans une pièce 8 formant un passage cylindrique 9 comprenant au moins un moyen d'aspersion 5 va brumiser une solution décontaminante sur toute la surface.

Selon un mode de réalisation préféré ladite solution décontaminante est choisie dans le groupe comprenant les liquides comprenant des dérivés de l'acridine, des dérivés de l'aluminium, des phénols et leurs dérivés, des dérivés du nitrofurane, des dérivés de l'iode, des dérivés de la quinoline, des Ammoniums quaternaires, des dérivés du mercure, du Peroxyde d'hydrogène, de l'éosine, du propanol, du tosylchloramide sodium, de l'isopropanol, du permanganate de potassium, de l'hypochlorite de sodium et de l'éthanol.

Selon un mode de réalisation préféré, le moyen d'aspersion 5 est relié à une enceinte contenant ladite solution décontaminante. Le passage de cette solution via le moyen d'aspersion va entrainer sa nébulisation et sa répartition sur toute la surface du matériel à décontaminer 2. La propulsion de la solution décontaminante à travers le moyen d'aspersion 5 est avantageusement réalisée grâce à une pompe.

Dans un deuxième temps, le matériel médical 2 va se déplacer verticalement, toujours en rotation sur lui-même dans la pièce 8 et passer devant le moyen de séchage 6 qui va permettre d'éliminer la solution décontaminante.

Ce moyen de séchage 6 est préférentiellement un moyen capable de produire une lame d'air. Il peut notamment prendre la forme d'une ouverture annulaire, présente à la surface interne de la pièce 8, avantageusement reliée à une source de gaz comprimé et encore plus avantageusement relié à la source d'air comprimé de l'établissement hospitalier recevant le dispositif selon l'invention. Préférentiellement, cette ouverture annulaire comporte deux lèvres 10, 10' capable d'orienter ladite lame d'air. Avantageusement, ladite lame d'air parcourt un trajet formant un angle compris entre 10° et 85° avec ledit axe prédéfini. Selon un mode de réalisation encore plus préféré, ledit angle est compris entre 20° et 50°.

Ensuite, le matériel médical 2 va se placer, pour un temps prédéfini, au centre de la pièce 8' comportant sur sa surface interne un moyen d'irradiation 7. Ledit moyen est préférentiellement une source de rayonnement d'UV-C.

Le matériel médical 2 revient alors en position nominale, prêt à être utilisé.

## Revendications

1. Dispositif (1) de décontamination pour matériel médical (2) comprenant un moyen d'aspersion (5), un moyen de séchage (6) et un moyen d'irradiation (7), ledit moyen d'aspersion (5), ledit moyen de séchage (6) et ledit moyen d'irradiation (7) sont compris dans une ou deux pièces, disposées selon le même axe longitudinal, formant un passage cylindrique, destiné à recevoir ledit matériel médical à décontaminer, le dispositif comprenant un support (3) destiné à recevoir et maintenir ledit matériel médical (2) à décontaminer selon l'axe de symétrie (4) longitudinal dudit passage cylindrique,
**caractérisé en ce que** ledit moyen d'aspersion (5) est monté en rotation autour dudit axe de symétrie (4) et en translation parallèlement audit axe de symétrie (4) de telle sorte que l'aspersion soit dirigée vers ledit axe de symétrie (4), **en ce que** ledit moyen de séchage (6) est un moyen capable de produire une lame d'air et est monté en translation parallèlement audit axe de symétrie (4), ladite lame d'air est destinée à parcourir un trajet formant un angle compris entre 10° et 85° avec ledit axe de symétrie (4), et **en ce que** ledit moyen d'irradiation est monté en translation parallèlement audit axe de symétrie (4).

2. Dispositif (1) de décontamination pour matériel médical (2) comprenant un moyen d'aspersion (5), un moyen de séchage (6) et un moyen d'irradiation (7), le dispositif comprenant un support (3) destiné à recevoir et maintenir ledit matériel médical (2) à décontaminer selon l'axe de symétrie (4) longitudinal dudit passage cylindrique, ledit support (3) est monté en rotation autour dudit axe de symétrie (4) et en translation selon ledit axe de symétrie (4), ledit moyen d'aspersion (5), ledit moyen de séchage (6) et ledit moyen d'irradiation (7) sont compris dans une ou deux pièces, disposées selon le même axe longitudinal, formant un passage cylindrique, destiné à recevoir ledit matériel médical à décontaminer,
**caractérisé en ce que** ledit moyen de séchage (6) est un moyen capable de produire une lame d'air et est monté en translation parallèlement audit axe de symétrie (4), ladite lame d'air est destinée à parcourir un trajet formant un angle compris entre 10° et 85° avec ledit axe de symétrie (4) .

3. Dispositif (1) de décontamination pour matériel médical (2) selon la revendication précédente **caractérisé en ce que** ledit moyen d'irradiation (7) forme un cylindre disposé sur la surface interne de la pièce (8, 8'), annulaire, dans laquelle il est compris.

4. Dispositif (1) de décontamination pour matériel médical (2) selon l'une des revendications précédentes **caractérisé en ce que** ledit moyen de séchage (6) est également monté en rotation autour de l'axe de symétrie (4) .

5. Dispositif (1) de décontamination pour matériel médical (2) selon l'une des revendications précédentes **caractérisé en ce que** ledit moyen d'irradiation (7) est également monté en rotation autour de l'axe de symétrie (4) .

6. Dispositif (1) de décontamination pour matériel médical (2) selon l'une des revendications précédentes **caractérisé en ce que** ledit moyen capable de produire une lame d'air est disposé annulairement autour de l'axe de symétrie (4).

7. Dispositif (1) de décontamination pour matériel médical (2) selon l'une des revendications précédentes **caractérisé en ce que** ledit moyen d'irradiation (7) est une source d'UV et préférentiellement d'UV-C.

8. Dispositif (1) de décontamination pour matériel médical (2) selon l'une des revendications précédentes **caractérisé en ce que** ledit moyen d'irradiation (7) est une source capable d'émettre séquentiellement ou simultanément des rayonnements appartenant à la gamme des UV-A, des UV-B et des UV-C.

9. Dispositif (1) de décontamination pour matériel médical (2) selon l'une des revendications précédentes **caractérisé en ce que** ledit moyen d'aspersion (5) est un moyen de nébulisation.

## Patentansprüche

1. Dekontaminationsvorrichtung (1) für medizinische Geräte (2) mit ein Spritz mittel (5), ein Trocknungsmittel (6) und ein Bestrahlungsmittel (7), wobei das Spritzmittel (5), das Trocknungsmittel (6) und das Bestrahlungsmittel (7) in einem oder zwei Teilen enthalten sind, die entlang derselben Längsachse angeordnet sind und einen zylindrischen Durchgang bilden, der dazu bestimmt ist, die zu dekontaminierenden medizinischen Geräte aufzunehmen, wobei die Vorrichtung einen Träger (3) umfasst, der dazu bestimmt ist, die zu dekontaminierenden medizinischen Geräte (2) entlang der Längssymmetrieachse (4) des zylindrischen Durchgangs aufzunehmen und zu halten, **dadurch gekennzeichnet, dass** das Spritzmittel (5) um die Symmetrieachse (4) drehbar und parallel zur Symmetrieachse (4) verschiebbar gelagert ist, so dass das Spritzen auf die Symmetrieachse (4) gerichtet ist, dass das Trocknungsmittel (6) ein Mittel ist, das einen Luftspalt erzeugen kann und parallel zur Symmetrieachse (4) verschiebbar angebracht ist, wobei der Luftspalt dazu bestimmt ist, eine Bahn zu durchlaufen, die einen Winkel zwischen 10° und 85° mit der Symmetrieachse (4) bildet, und dass das Bestrahlungsmittel parallel zur Symmetrieachse (4) verschiebbar angebracht ist.

2. Dekontaminationsvorrichtung (1) für medizinische Geräte (2), die ein Spritzmittel (5), ein Trocknungsmittel (6) und ein Bestrahlungsmittel (7) umfasst, wobei die Vorrichtung einen Träger (3) umfasst, der dazu bestimmt ist, die zu dekontaminierenden medizinischen Geräte (2) entlang der Längssymmetrieachse (4) des zylindrischen Durchgangs aufzunehmen und zu halten, wobei der Träger (3) um die Symmetrieachse (4) drehbar und entlang der Symmetrieachse (4) verschiebbar angebracht ist, wobei das Spritzmittel (5), das Trocknungsmittel (6) und das Bestrahlungsmittel (7) in einem oder zwei Teilen enthalten sind, die entlang derselben Längsachse angeordnet sind und einen zylindrischen Durchgang bilden, der dazu bestimmt ist, die zu dekontaminierenden medizinischen Geräte aufzunehmen, **dadurch gekennzeichnet, dass** das Trocknungsmittel (6) ein Mittel ist, das einen Luftspalt erzeugen kann und parallel zur Symmetrieachse (4) verschiebbar angebracht ist, wobei der Luftspalt dazu bestimmt ist, eine Bahn zu durchlaufen, die mit der Symmetrieachse (4) einen Winkel zwischen 10° und 85° bildet.

3. Dekontaminationsvorrichtung (1) für medizinische Geräte (2) nach dem vorhergehenden Anspruch, **dadurch gekennzeichnet, dass** das Bestrahlungsmittel (7) einen Zylinder bildet, der auf der Innenfläche des ringförmigen Teils (8, 8') angeordnet ist, in dem er enthalten ist.

4. Dekontaminationsvorrichtung (1) für medizinische Geräte (2) nach einem der vorhergehenden Ansprüche, **dadurch gekennzeichnet, dass** das Trocknungsmittel (6) auch um die Symmetrieachse (4) drehbar gelagert ist.

5. Dekontaminationsvorrichtung (1) für medizinische Geräte (2) nach einem der vorhergehenden Ansprüche, **dadurch gekennzeichnet, dass** das Bestrahlungsmittel (7) auch um die Symmetrieachse (4) drehbar gelagert ist.

6. Dekontaminationsvorrichtung (1) für medizinische Geräte (2) nach einem der vorhergehenden Ansprüche, **dadurch gekennzeichnet, dass** das Mittel, das einen Luftspalt erzeugen kann, ringförmig um die Symmetrieachse (4) angeordnet ist.

7. Dekontaminationsvorrichtung (1) für medizinische Geräte (2) nach einem der vorhergehenden Ansprüche, **dadurch gekennzeichnet, dass** das Bestrahlungsmittel (7) eine UV- und vorzugsweise eine UV-C-Quelle ist.

8. Dekontaminationsvorrichtung (1) für medizinische Geräte (2) nach einem der vorhergehenden Ansprüche, **dadurch gekennzeichnet, dass** das Bestrahlungsmittel (7) eine Quelle ist, die in der Lage ist, sequentiell oder gleichzeitig Strahlung zu emittieren, die zum UV-A-, UV-B- und UV-C-Bereich gehört.

9. Dekontaminationsvorrichtung (1) für medizinische Geräte (2) nach einem der vorhergehenden Ansprüche, **dadurch gekennzeichnet, dass** das Spritzmittel (5) eine Vernebelungmittel ist.

## Claims

1. Decontamination device (1) for medical material (2), comprising a spraying means (5), a drying means (6) and an irradiating means (7), said spraying means (5), said drying means (6) and said irradiating means (7) are included in one or two parts, provided along the same longitudinal axis, forming a cylindrical passage, intended to receive said medical material to be decontaminated, the device comprising a support (3) intended to receive and hold said medical material (2) to be decontaminated along the longitudinal axis of symmetry (4) of said cylindrical passage, **characterised in that** said spraying means (5) is mounted in rotation about said axis of symmetry (4) and in translation parallel to said axis of symmetry (4) in such a way that the spraying is directed towards said axis of symmetry (4), **in that** said drying means (6) is a means capable of producing an air knife and is mounted in translation parallel to said axis of symmetry (4), said air knife being intended to pass through a trajectory forming an angle between 10° and 85° with said axis of symmetry (4), and **in that** said irradiating means is mounted in translation parallel to said axis of symmetry (4).

2. Decontamination device (1) for medical material (2), comprising a spraying means (5), a drying means (6) and an irradiating means (7), the device comprising a support (3) intended to receive and hold said medical material (2) to be decontaminated (4) along the longitudinal axis of symmetry (4) of said cylindrical passage, said support (3) is mounted in rotation about said axis of symmetry (4) and in translation on said axis of symmetry (4), said spraying means (5), said drying means (6) and said irradiating means (7) are included in one or two parts, provided along the same longitudinal axis, forming a cylindrical passage, intended to receive said medical material to be decontaminated, **characterized in that** said drying means (6) is a means capable of producing an air knife and is mounted in translation parallel to said axis of symmetry (4), said air knife being intended to pass through a trajectory forming an angle between 10° and 85° with said axis of symmetry (4).

3. Decontamination device (1) for medical material (2) according to the preceding claim, **characterised in that** said irradiating means (7) forms a cylinder arranged on the inner surface of the part (8, 8'), which is annular, in which it is included.

4. Decontamination device (1) for medical material (2) according to any of the preceding claims, **characterised in that** said drying means (6) is also mounted in rotation about the axis of symmetry (4).

5. Decontamination device (1) for medical material (2) according to any of the preceding claims, **characterised in that** said irradiating means (7) is also mounted in rotation about the axis of symmetry (4).

6. Decontamination device (1) for medical material (2) according to any of the preceding claims, **characterised in that** said means capable of producing an air knife is arranged annularly about the axis of symmetry (4) .

7. Decontamination device (1) for medical material (2) according to any of the preceding claims, **characterised in that** said irradiating means (7) is a source of UV and preferentially of UV-C.

8. Decontamination device (1) for medical material (2) according to any of the preceding claims, **characterised in that** said irradiating means (7) is a source capable of emitting sequentially or simultaneously radiation belonging to the UV-A, UV-B and UV-C range.

9. Decontamination device (1) for medical material (2) according to any of the preceding claims, **characterised in that** said spraying means (5) is a nebulisation means.
